# EUROPEAN PATENT SPECIFICATION

(11) **EP 3 684 924 B1**
(45) Date of publication and mention of the grant of the patent: **02.11.2022**
(21) Application number: 18769377.5
(22) Date of filing: 19.09.2018
(51) Int. Cl.: C12N 15/00

(54) **NON-INTEGRATING DNA VECTORS FOR THE GENETIC MODIFICATION OF CELLS**
NICHT INTEGRIERENDE DNA-VEKTOREN ZUR GENETISCHEN MODIFIKATION VON ZELLEN
VECTEURS D'ADN NON INTÉGRANTS DESTINÉS À LA MODIFICATION GÉNÉTIQUE DE CELLULES

(30) Priority: 19.09.2017 EP 17191829
(43) Date of publication of application: 29.07.2020
(73) Proprietor: Deutsches Krebsforschungszentrum, 69120 Heidelberg (DE)
(72) Inventor: HARBOTTLE, Richard, 68526 Ladenburg (DE); BOZZA, Matthias, 69121 Heidelberg (DE); WILLIAMS, James A., Lincoln, Nebraska 68516 (US)
(74) Representative: Altmann Stößel Dick Patentanwälte PartG mbB
(86) International application number: PCT/EP2018/075353
(87) International publication number: WO 2019/057773

(56) References cited:
- WO-A1-2010/006215
- WO-A1-2014/016580
- WO-A2-2010/018444
- US-A- 5 985 607
- ANJA EHRHARDT ET AL: "Episomal Vectors for Gene Therapy", CURRENT GENE THERAPY, vol. 8, no. 3, 1 June 2008 (2008-06-01), pages 147-161, XP055436780, NL ISSN: 1566-5232, DOI: 10.2174/156652308784746440
- ZIQING LIU ET AL: "Systematic comparison of 2A peptides for cloning multi-genes in a polycistronic vector", SCIENTIFIC REPORTS, vol. 7, no. 1, 19 May 2017 (2017-05-19), XP055515282, DOI: 10.1038/s41598-017-02460-2
- WEST A G ET AL: "Insulators: Many functions, many mechanisms", GENES AND DEVELOPMENT, COLD SPRING HARBOR LABORATORY PRESS, PLAINVIEW, NY, US, vol. 16, no. 3, 1 February 2002 (2002-02-01), pages 271-288, XP002249349, ISSN: 0890-9369, DOI: 10.1101/GAD.954702
- S. C. VERGHESE ET AL: "S/MAR sequence confers long-term mitotic stability on non-integrating lentiviral vector episomes without selection", NUCLEIC ACIDS RESEARCH, 27 January 2014 (2014-01-27), XP055102696, ISSN: 0305-1048, DOI: 10.1093/nar/gku082

## Description

The present disclosure relates to a polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA (SEQ ID NO:1) per 100 nucleotides over a stretch of at most 200 nucleotides; the present disclosure further relates to a composition and to a host cell comprising said polynucleotide, and to the polynucleotide for use in medicine and for use in treating genetic disease. The present disclosure also relates to a kit and to a device comprising said polynucleotide, and to methods and uses related to the polynucleotide.

Genetic modification of cells is used routinely in modern cell culture for scientific purposes. However, use of corresponding techniques in treatment of inherited diseases caused by mutations of genes, while being highly desirable, still is hampered by the problem that methods available usually only provide transient modification, such as transient transfection protocols, whereas methods providing stable modification of cells usually rely on integration of the transgene into the genome of the host cell. Integration of a transgene, however, even if targeted to a specific locus, bears the risk of inducing a deleterious mutation, which may lead e.g. to cancer as a side effect of treatment.

Scaffold/matrix attachment regions (S/MARs), which are also known as scaffold-attachment regions (SARs) or matrix-associated regions (MARs) are known as sequences in the genome of eukaryotic organisms mediating attachment of the nuclear matrix. The S/MARS are AT-rich sequences, and some AT-rich motifs were found to be further enriched (Liebeich et al. (2002), NAR 30(15): 3433). A variety of vectors has been proposed for stable maintenance in cells based on S/MAR motifs, e.g. in US 6,410,314 B1, US 5,985,607 A, WO 2010/018444 A2, and in Haase et al. (2010), BMC Biotechnology 10:20; moreover, epigenetic effects having an influence on replication of such vectors were identified (Haase et al.(2013), PLOS One 8(11):e79262). Nonetheless, S/MAR-based vectors being stable enough for use in gene therapy are still not available.

There is, nonetheless, a need in the art for improved means and methods for stable transfection of cells, in particular using S/MAR elements and avoiding the risks involved with integration of the transgene into the genome of the host cell. This problem is solved by the means and methods disclosed herein.

Accordingly, the present invention relates to a polynucleotide according to claim 1; further disclosure relates to a polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA per 100 nucleotides over a stretch of at most 200 nucleotides.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The term "polynucleotide", as used herein, refers to a linear or circular nucleic acid molecule. The term encompasses single as well as partially or completely double-stranded polynucleotides. Preferably, the polynucleotide is RNA or is DNA, including cDNA. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention shall be provided, preferably, either as an isolated polynucleotide (i.e. isolated from its natural context) or in genetically modified form. The polynucleotide of the invention comprises at least one promoter active in a host cell and an S/MAR element; moreover, the polynucleotide has the biological activity of replicating episomally in a host cell, all as specified herein below. Preferably, the polynucleotide has a length of at most 1 Mb, more preferably at most 500 kb, even more preferably at most 200 kb, most preferably at most 100 kb. Preferably, the polynucleotide is a non-naturally occurring polynucleotide; thus, preferably, the nucleotide is an artificial polynucleotide. Also preferably, the polynucleotide is a chimeric polynucleotide; more preferably, the polynucleotide comprises at least one nucleic acid sequence heterologous to the remaining nucleic acid sequences it comprises.

As used herein, the term polynucleotide, preferably, includes variants of the specifically indicated polynucleotides. More preferably, the term polynucleotide relates to the specific polynucleotides indicated. The term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the biological activity or activities as specified for the specific polynucleotide. Thus, it is to be understood that a polynucleotide variant as referred to in accordance with the present invention shall have a nucleic acid sequence which differs due to at least one nucleotide substitution, deletion and/or addition. Preferably, said polynucleotide variant comprises an ortholog, a paralog or another homolog of the specific polynucleotide or of a functional subsequence thereof, e.g. of an S/MAR element. Also preferably, said polynucleotide variant comprises a naturally occurring allele of the specific polynucleotide or of a functional subsequence thereof. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in standard textbooks A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of O.lx to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G+C content of 50% in the absence of formamide; accordingly, other conditions more suitable for low-G+C DNA, which are in principle known to the skilled person, may be found to be more appropriate by the skilled person. The skilled worker knows how to determine the hybridization conditions required by referring to standard textbooks. Alternatively, polynucleotide variants are obtainable by PCR-based techniques such as mixed oligonucleotide primer- based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequences thereof. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), are preferably used. Preferably, said programs are used with their standard parameters. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide of the present invention. A fragment as meant herein, preferably, comprises at least 200, preferably at least 300, more preferably at least 400 consecutive nucleotides of any one of the specific nucleic acid sequences; or encodes an amino acid sequence comprising at least 100, preferably at least 200, more preferably at least 300 consecutive amino acids of any one of the specific amino acid sequences and still having the indicated activity.

The polynucleotides of the present invention either consist, essentially consist of, or comprise the aforementioned nucleic acid sequences. Thus, they may contain further nucleic acid sequences as well. Specifically, the polynucleotides of the present invention may encode e.g. fusion proteins or selectable markers. Such fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein.

Also preferably, the polynucleotide comprises at least one cargo sequence. The term "cargo sequence", as used herein, relates to a nucleic acid sequence of interest of being transferred into and stably maintained in a host cell. Preferably, the cargo sequence is a nucleic acid sequence encoding a polynucleotide, e.g. an RNA, and/or a polypeptide of interest. Preferably, the polypeptide of interest is a therapeutic polypeptide, more preferably a T Cell Receptor (TCR), more preferably a human or chimeric T Cell receptor, a Chimeric Antigen Receptor (CAR), preferably MARTI TCR, or a polypeptide lacking in cells affected with a genetic disease as specified elsewhere herein. Thus, e.g. preferably, the polynucleotide comprises at least one cargo sequence encoding a polypeptide providing phenylalanine-hydroxylase activity (EC 1.14.16.1) for treatment of phenylketonuria. In a preferred embodiment, the cargo sequence intervenes the at least one promoter and the S/MAR element. In a further preferred embodiment, the cargo sequence intervenes the at least one promoter and the S/MAR element, wherein said S/MAR element is flanked by a splice donor and a splice acceptor; thus, preferably, the S/MAR element is spliced out from a transcript encoding the cargo sequence. Thus, in a preferred embodiment, the polynucleotide further comprises a coding sequence encoding a polypeptide, wherein said coding sequence encoding a polypeptide intervenes said promoter and said S/MAR element. In a further preferred embodiment, the sequence encoding a polypeptide intervenes the at least one promoter and the S/MAR element, wherein said S/MAR element is flanked by a splice donor and a splice acceptor; thus, preferably, the S/MAR element is spliced out from a transcript encoding the polypeptide.

Preferably, the sequence encoding a selectable marker and the cargo sequence are intervened by a sequence enabling expression of two (or more) polypeptides in a eukaryotic cell from one mRNA, e.g. an internal ribosomal entry sequence (IRES) or, more preferably, a self-cleaving peptide sequence such as, most preferably, a peptide 2A (P2A) sequence from porcine teschovirus-1. Appropriate sequences are known in the art, e.g. from Kim et al. (2011) PLoS ONE 6(4): e18556.

Preferably, the polynucleotide is a DNA. Preferably, the polynucleotide comprises further expression control sequences allowing expression of genes in prokaryotic and/or eukaryotic, preferably in eukaryotic host cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Examples for regulatory elements permitting expression in eukaryotic host cells are the AOX1 or GAL1 promoter in yeast or the SMVP-, U6-, H1I-, 7SK-, CMV- EFS-, SV40-, or RSV-promoter (Rous sarcoma virus), CMV-enhancer, SV40-enhancer or a globin intron in mammalian and other animal cells. Moreover, inducible or cell type-specific expression control sequences may be comprised in a polynucleotide of the present invention. Inducible expression control sequences may comprise tet or lac operator sequences or sequences inducible by heat shock or other environmental factors. Suitable expression control sequences are well known in the art. Besides elements which are responsible for the initiation of transcription, such regulatory elements may also comprise transcription termination signals, such as the SV40-poly-A site or the tk-poly-A site, downstream of the polynucleotide.

The term "host cell", as used herein, relates to any cell capable of receiving and stably replicating the polynucleotide. Preferably, the host cell is a eukaryotic cell, preferably a plant or yeast cell, e.g. a cell of a strain of baker's yeast, or is an animal cell. More preferably, the host cell is an insect cell or a mammalian cell, in particular a mouse or rat cell. Even more preferably, the host cell is a mammalian cell, most preferably is a human cell. Preferably, the host cell is a CD34+ Progenitor Cell; a CD61+ Thrombocyte; a CD19+ B-Lymphocyte; a CD14+ Monocyte; a CD15+ Granulocyte; a CD3+ Cytotoxic T-Lymphocyte, preferably also positive for CD8 and CD45; a CD3+ Helper T-Lymphocyte, preferably also positive for CD4 and CD45; a CD3+ activated T-Lymphocyte, preferably also positive for CD25 and CD45, a Tumor infiltrating Lymphocyte, or a Natural Killer (NK) cell. As will be understood by the skilled person, the polynucleotide may in addition have sequences permitting replication in a bacterial cell, in particular a bacterial origin of replication. Preferably, the bacterial cell is a cell of a laboratory strain of bacteria, more preferably an Escherichia coli cell.

The term "promoter" is, in principle, known to the skilled person as a genetic element directing, optionally in concert with further regulatory elements, the level of transcription of a given gene. A promoter may be constitutive, i.e. providing a constant level of transcription essentially independent of a host cell's state, or may be regulated, i.e. provide levels of transcription in dependence of a host cell's state. Moreover, a promoter may be cell type and/or tissue specific, i.e. provide a detectable level of transcription only in a few or only one cell type. Preferably, the promoter according to the present invention is active in the host cell as specified herein above. As will be understood by the skilled person, the selection of promoter may depend on the type of host cell intended for targeting; suitable promoters for specific cell types as well as constitutive promoters are known in the art. Preferably, the promoter is a eukaryotic promoter, more preferably a constitutive eukaryotic promoter, even more preferably a strong eukaryotic promoter. Preferably, the promoter is an EF1alpha (elongation factor 1 alpha) promoter, an UbiC (ubiquitin C) promoter, a ROSA 26 promoter, a PGK (phosphoglycerate kinase) promoter, and/or a CAG (chicken alpha-actin) promoter, more preferably is an EF1alpha promoter. Also preferably, the promoter is a cell- and/or tissue-specific eukaryotic promoter. As used herein, the term "promoter" is used for the promoter as specified above, whereas any other promoter potentially present on the polynucleotide in addition is referred to as "secondary promoter". Thus, preferably, the promoter is a promoter directing transcription into the S/MAR sequence in a host cell; also preferably, a promoter not directing transcription into the S/MAR sequence of the polynucleotide, e.g. being a prokaryotic promoter, being transcriptionally insulated from the S/MAR sequence, and/or being a promoter directing transcription away from the S/MAR sequence, is a secondary promoter. Preferably, the promoter comprises less than 1000, more preferably less than 250, even more preferably less than 100, most preferably less than 20 contiguous base pairs corresponding to an Apolipoprotein B promoter; thus, preferably, the polynucleotide does not comprise a human Apolipoprotein B promoter, more preferably does not comprise an Apolipoprotein B promoter.

Preferably, the S/MAR sequence is located immediately downstream of the promoter and, if present, of the selectable marker gene as specified herein below. Preferably, being located "immediately downstream" is lacking an intervening transcription termination signal, more preferably is lacking an intervening gene. Thus, preferably, transcripts initiated at the promoter and, if encoded, including the detectable marker sequence preferably comprise a transcribed S/MAR sequence, more preferably comprise the complete S/MAR sequence comprised in the polynucleotide; as will be understood by the skilled person in view of the description elsewhere herein, the polynucleotide may further include splicing sites mediating excision of the S/MAR sequence from the primary transcript; thus, more preferably, preferably, at least primary transcripts initiated at the promoter and, if encoded, including the detectable marker sequence preferably comprise a transcribed S/MAR sequence, more preferably comprise the complete S/MAR sequence comprised in the polynucleotide. Also preferably, the term "immediately downstream" includes a polynucleotide in which the promoter and the S/MAR sequence are separated by elongated nucleic acid sequences, provided a transcription termination signal is not intervening the promoter and the S/MAR. Preferably, the sequence intervening the promoter or, if present, the stop codon of the selectable marker gene and the S/MAR sequence has a length of at most 2 kb, more preferably at most 0.5 kb, even more preferably at most 0.2 kb, still more preferably at most 0.1 kb, most preferably at most 50 bp.

The term "S/MAR element", also known under the designation "scaffold/matrix attachment region", is, in principle, known to the skilled person to relate to a DNA sequence mediating attachment of the nuclear matrix of a eukaryotic cell to said DNA. S/MAR sequences typically are derived from sequences in the DNA of eukaryotic chromosomes. A variety of S/MAR sequences is available, and sequences are available from public databases, e.g. as described in Liebich et al. (2002), Nucleic Acids Res. 30, 312-374. According to the present invention, the nucleic acid sequence of said S/MAR element (hitherto referred to as S/MAR sequence) comprises at least 3 sequence motifs ATTA per 100 nucleotides over a stretch of at most 200 nucleotides. Thus, the motif comprised in the S/MAR sequence comprises a multitude of the four-nucleotide motif 5'-ATTA-3'. Preferably, the S/MAR sequence has a length of at least 200 nucleotide, more preferably at least 300 nucleotides, even more preferably at least 400 nucleotides, most preferably at least 500 nucleotides. Preferably, the S/MAR sequence has a length of at most 3 kb, more preferably at most 2 kb, even more preferably at most 1.5 kb, still more preferably at most 1 kb, most preferably at most 0.9 kb. In a preferred embodiment, the S/MAR sequence has a length of at most 0.7 kb, more preferably at mot 500 bp, most preferably at most 250 bp. Thus, preferably, the S/MAR sequence has a length of from 0.2 kb to 3 kb, more preferably of from 0.3 kb to 2 kb, even more preferably of from 0.4 kb to 1.5 kb, most preferably of from 0.5 kb to 1 kb. As will be understood, the indication "comprises n sequence motifs per 100 nucleotides" relates to the average number of said sequence motifs calculated per 100 base pairs of sequence and, accordingly, may be a fraction number. E.g. the number of ATTA sequence motifs per 100 base pairs in SEQ ID NO:6 is 34 / 525 base pairs ^{∗} 100 base pairs = 6.5. Preferably, the number of sequence motifs per 100 base pairs is determined over the whole length of the S/MAR sequence; in case of doubt, e.g. where a boundary of the S/MAR sequence cannot be determined, the number of sequence motifs per 100 base pairs of a polynucleotide, preferably, is the highest number determinable for any window of 200 bp within said polynucleotide, more preferably is the highest number determinable for any window of 500 bp within said polynucleotide. Preferably, the S/MAR sequence comprises at least 4 sequence motifs ATTA per 100 nucleotides over a stretch of at most 200 nucleotides, more preferably at least 5 sequence motifs ATTA per 100 nucleotides over a stretch of at most 200 nucleotides, still more preferably at least 6 sequence motifs ATTA per 100 nucleotides over a stretch of at most 200 nucleotides. Also preferably, the S/MAR sequence comprises at least 3 sequence motifs ATTA per 100 nucleotides over a stretch of at most 400 nucleotides, more preferably at least 4 sequence motifs ATTA per 100 nucleotides over a stretch of at most 400 nucleotides, even more preferably at least 5 sequence motifs ATTA per 100 nucleotides over a stretch of at most 400 nucleotides, most preferably at least 6 sequence motifs ATTA per 100 nucleotides over a stretch of at most 400 nucleotides. Also preferably, the S/MAR sequence comprises at least 3 sequence motifs ATTA per 100 nucleotides over a stretch of at most 500 nucleotides, more preferably at least 4 sequence motifs ATTA per 100 nucleotides over a stretch of at most 500 nucleotides, still more preferably at least 5 sequence motifs ATTA per 100 nucleotides over a stretch of at most 500 nucleotides, most preferably at least 6 sequence motifs ATTA per 100 nucleotides over a stretch of at most 500 nucleotides. Thus, preferably, the S/MAR sequence comprises at least 10 sequence motifs ATTA over a sequence of 500 nucleotides, more preferably at least 20 sequence motifs ATTA over a sequence of 500 nucleotides, still more preferably at least 30 sequence motifs ATTA over a sequence of 500 nucleotides. Preferably, at least 80%, more preferably at least 90%, most preferably at least 95% of the ATTA motifs in the S/MAR sequence are separated by of from 9 to 13, preferably by 10 to 12, most preferably by 11 base pairs, respectively.

Preferably, the S/MAR element comprises additional sequence motifs, preferably within the sequence comprising the ATTA motifs described herein above. Preferably, the sequence stretch of said S/MAR element comprising said ATTA sequence motifs further comprises at least one sequence motif ATTTA (SEQ ID NO:2), preferably at least 2 sequence motifs ATTTA, more preferably at least 4 sequence motifs ATTTA, most preferably at least 8 sequence motifs ATTTA. Also preferably, the sequence stretch of said S/MAR element comprising said ATTA sequence motifs and, optionally, said ATTTA motif(s), further comprises at least one, preferably at least two, more preferably at least four, most preferably at least six palindromic motifs, preferably motifs TAAATATTTTA (SEQ ID NO:3). Preferably, said motifs TAAATATTTTA are contiguous with at least one motif ATTA on the 5' end and/or the 3' end. Also preferably, the sequence stretch of the S/MAR element comprising said ATTA sequence motifs comprises at least one, preferably at least two, more preferably at least three, even more preferably at least four, most preferably at least five sequence motifs ATTATAAATATTTTAATTA (SEQ ID NO:4), more preferably sequence motifs ATTTAATTATAAATATTTTAATTA (SEQ ID NO:5).

Also preferably, the S/MAR sequence has a low G+C content. The skilled person knows how to calculate the C+G content of a known sequence by counting all guanine and cytidine bases in the sequence and dividing the cumulated result by the number of nucleotides in the sequence. Preferably, the sequence stretch of the S/MAR element comprising said sequence motifs ATTA has a G+C content of at most 30%, more preferably at most 20%, still more preferably at most 15%, even more preferably at most 10%, most preferably at most 5%. Preferably, in cases where the boundary of an S/MAR element cannot be determined, the sequence used for calculation of the G+C content is the same used for calculating the number of ATTA motifs per 100 base pairs, as specified herein above. Also preferably, the S/MAR sequence has a low number of CG dinucleotides. Preferably, the sequence stretch of said S/MAR element comprising said sequence motifs comprises at most 6 sequence motifs CG, more preferably at most 4, even more preferably at most 2, most preferably does not comprise a sequence motif CG.

Preferably, the S/MAR sequence comprises an S/MAR sequence of an Apolipoprotein B gene, preferably a human Apolipoprotein B gene, more preferably a 3' S/MAR sequence of a human Apolipoprotein B gene. More preferably, the S/MAR sequence comprises a variant of a human Apolipoprotein B gene, more preferably of a 3' S/MAR sequence of a human Apolipoprotein B gene. Thus, preferably, the S/MAR sequence comprises a sequence at least 70% identical to the sequence of SEQ ID NO:6, preferably of SEQ ID NO:7 or 8. More preferably, the S/MAR sequence comprises the nucleic acid sequence of SEQ ID NO:6, preferably of SEQ ID NO:7, more preferably SEQ ID NO:8. In a preferred embodiment, the S/MAR sequence comprises a sequence at least 70% identical to the sequence of SEQ ID NO: 15, more preferably the S/MAR sequence comprises the sequence of SEQ ID NO: 15.

Preferably, the polynucleotide comprises a poly-A signal downstream of the S/MAR element: More preferably, the polynucleotide comprises a poly-A signal and a transcription termination signal downstream of the S/MAR element. Also preferably, the S/MAR element is flanked by a splice donor and a splice acceptor; thus, in a preferred embodiment, a transcript is transcribed from the promoter, from which transcript the sequence of the S/MAR element is spliced out. Also preferably, the S/MAR sequence preferably is spliced out of the transcript encoding the selectable marker after transcription. Also preferably, the polynucleotide further comprises a (secondary) bacterial origin of replication as specified herein above and/or a bacterial selectable marker gene. Preferably, the bacterial origin of replication and the promoter driving expression of the bacterial selectable marker gene are prokaryote-specific, i.e., more preferably, are non-functional in a host cell. Also preferably, the bacterial origin of replication and/or bacterial selectable marker gene, preferably all elements active in a prokaryotic cell comprised in the polynucleotide, is/are insulated from the residual sequences comprised in the polynucleotide by the presence of at least one insulation element, more preferably by being flanked by insulation elements. Preferably, the bacterial origin of replication and/or bacterial selectable marker gene, preferably all elements active in a prokaryotic cell, is/are insulated from the residual sequences comprised in the polynucleotide by the presence of at least one insulating element at the 5' end and of at least one insulating element at the 3' end. More preferably, the bacterial origin of replication and/or bacterial selectable marker gene, preferably all elements active in a prokaryotic cell comprised in the polynucleotide, is/are insulated from the promoter by the presence of at least one insulation element, more preferably by being flanked by insulation elements. Preferably, said insulation element(s) is(are) an anti-repressive element40 element (SEQ ID NO:11) or a variant thereof and/or an S/MAR element.

Thus, preferably, the polynucleotide comprises the sequence of SEQ ID NO:7 or 8 or of a sequence at least 70% identical to the sequence of SEQ ID NO:7 or 8; preferably of SEQ ID NO:12 or of a sequence at least 70% identical to the sequence of SEQ ID NO:12, more preferably of SEQ ID NO:13 or of a sequence at least 70% identical to the sequence of SEQ ID NO:13, most preferably of SEQ ID NO:14 or of a sequence at least 70% identical to the sequence of SEQ ID NO:14. Preferably, the polynucleotide comprises the sequence of SEQ ID NO:14 with the nucleic acid sequence encoding GFP replaced by a nucleic acid sequence encoding a different polypeptide, preferably a therapeutic polypeptide, more preferably human T Cell Receptor (TCR), Chimeric Antigen Receptor (CAR), preferably MARTI TCR.

Preferably, the polynucleotide further comprises a coding sequence encoding a selectable marker polypeptide, said selectable marker sequence intervening the promoter of the polynucleotide and the S/MAR element of the polynucleotide, preferably wherein said promoter and said selectable marker sequence together constitute a selectable marker gene. As used herein, the term "selectable marker sequence" is used as a shorthand for the expression "coding sequence encoding a selectable marker polypeptide". The term "selectable marker" is in principle understood by the skilled person and relates to a nucleic acid sequence conferring, when expressed in a host cell, resistance to at least one condition mediating selective pressure to a host cell when applied thereto. Selectable markers are known in the art for prokaryotic and for eukaryotic cells. Preferably, the selectable marker is a selectable marker of an eukaryotic cell. Preferably, the selectable marker is a selectable marker polypeptide, more preferably a selectable marker polypeptide having transporter and/or enzymatic activity removing a selective compound from a host cell or modifying said selective compound to make it inactive. Preferably, the selectable marker gene further encodes at least one intron, preferably upstream of the sequence encoding the selectable marker polypeptide. Preferably, the selectable marker is a marker mediating resistance to puromycin, to blasticidin, neomycin, and/or to zeocin, more preferably to puromycin. Thus, preferably, the promoter and the selectable marker together constitute a puromycin resistance gene, a blasticidin resistance gene, a neomycin resistance gene, or a zeocin resistance gene, more preferably a puromycin resistance gene. In a preferred embodiment, the selectable marker is a polypeptide providing resistance to a specific set of growth conditions, preferably presence and/or absence of proliferation signals. Thus, in a preferred embodiment, the selectable marker is a T-cell receptor (TCR) or a chimeric antigen receptor (CAR), both of which are in principle known in the art. Preferably, the TCR and/or the CAR have a known specificity, such that, preferably, T cell signaling can be induced in host cells comprising said TCR and/or CAR. Preferably, in such case, the host cell is a T cell or an NK cell. Preferably, the selectable marker gene is devoid of a poly-A signal and of transcription termination signal(s). Thus, in a preferred embodiment, the polynucleotide further comprises a coding sequence encoding a selectable marker (selectable marker sequence), said selectable marker sequence intervening said promoter and said S/MAR element, wherein said promoter and said selectable marker sequence together constitute a selectable marker gene, and wherein said selectable marker is a selectable marker of a eukaryotic cell

Preferably the selectable marker is the puromycin acetyltransferase (Genbank Acc No. KX548903.1 (SEQ ID NO:9), encoded by nucleotides 535 to 1134 of Genbank Acc No. KX548903.1 (SEQ ID NO:10)). Thus, the selectable marker gene, preferably, comprises a nucleic acid sequence which a) causes expression of a puromycin resistance polypeptide comprising the sequence of SEQ ID NO:9; b) causes expression of a puromycin resistance polypeptide comprising a sequence at least 70% identical to the sequence of SEQ ID NO:9; c) comprises the sequence of SEQ ID NO:10; d) comprises a sequence at least 70% identical to the sequence of SEQ ID NO:10, e) comprises a nucleic acid sequence encoding a puromycin resistance polypeptide comprising, preferably consisting of, the sequence of SEQ ID NO:9, and/or f) comprises a nucleic acid sequence encoding a puromycin resistance polypeptide comprising, preferably consisting of, a sequence at least 70% identical to the sequence of SEQ ID NO:9.

As used herein, the term "replicating" relates to the activity of the polynucleotide to induce production of at least two replicas of said polynucleotide in a host cell during a cell replication cycle. Thus, preferably, replication of a polynucleotide in a host cell is determined by determining the presence of the polynucleotide after a series of cell divisions, in which a non-replicating polynucleotide would have been expected to be diluted out. Preferably, replication is stable replication, i.e. is replication to such an extent that the polynucleotide still is detectable in a host cell population after on average 50 cell divisions, more preferably after on average 100 cell divisions, most preferably after on average 250 cell divisions. Preferably, detection of a polynucleotide in a host cell population is performed by PCR under standard conditions.

The term "episomal" replication is, in principle, known to the skilled person to relate to replication of a polynucleotide without being integrated into the cellular genome, i.e. without becoming covalently attached to the cellular genome. Thus, preferably, episomal replication of a polynucleotide is replication of said polynucleotide as an autonomous replication unit. Preferably, episomal replication is maintenance of the polynucleotide in the host cell in the form of a circularly closed double-stranded DNA molecule. As will be understood by the skilled person, the actual replication of said polynucleotide may involve other forms, e.g. in rolling circle replication. Episomal maintenance of circular DNA preferably is verified by the plasmid rescue procedure known to the skilled person; i.e. preferably, by preparing a lysate of host cells and transforming the DNA comprised therein into appropriate bacterial cells, e.g. E. coli cells; if a suitable number of bacterial colonies obtainable by said method comprises the circular DNA as a plasmid having the same restriction pattern and/or sequence as the original circular DNA, it is, preferably, assumed that the circular DNA was maintained episomally. A further method of verifying episomal maintenance, which is also known to the skilled person, is DNA/DNA blotting ("Southern Blot" method); thus, preferably, total DNA of host cells is prepared and digested with one or more restriction enzyme(s); if in a Southern Blot using the original plasmid as a probe only bands corresponding to the original circular DNA are visible, it is preferably concluded that the plasmid is maintained episomally. More preferably, episomal maintenance is verified as described herein in the Examples.

In accordance, the term "replicating episomally", as used herein, relates to the activity of a polynucleotide to induce production of at least two replicas of said polynucleotide in a host cell during a cell replication cycle while said polynucleotide is present in said cell as an autonomously replicating entity; and stable episomal replication is episomal replication to such an extent that the polynucleotide is still detectable in the host cell after at least 50 cell divisions, preferably after at least 100 cell divisions, more preferably, after at least 250 cell divisions, most preferably, after at least 500 cell divisions. Preferably, the aforesaid number of cell divisions is the average number of cell divisions for a population of cells.

The polynucleotide of the present invention preferably is devoid of a of a simian virus 40 (SV40) origin of replication, a bovine papillomavirus (BPV) origin of replication, and an Epstein-Barr virus (EBV) origin of replication, preferably is devoid of a polyomavirus origin of replication, a papillomavirus origin of replication, and a herpesvirus origin of replication; more preferably is devoid of an origin of replication of an eukaryote-infecting virus. More preferably, the vector is devoid of any known eukaryotic origin of replication. However, preferably, the polynucleotide further comprises a prokaryotic, preferably a bacterial origin of replication, in particular an E. coli origin of replication. Preferably, the prokaryotic origin of replication is the only origin of replication comprised in the polynucleotide.

Advantageously, it was found in the work underlying the present invention that by combining an S/MAR element as specified with a promoter reading into said S/MAR element, a polynucleotide is obtained which is highly stable in episomal form in host cells, even in the absence of a dedicated origin of replication. Moreover, it was found that efficacy of establishment of the polynucleotide could be further improved by using a puromycin resistance gene, by ensuring transcription into the S/MAR element through the resistance gene, and by insulating the promoter - S/MAR combination transcriptionally from other promoters potentially present in the polynucleotide.

The definitions made above apply mutatis mutandis to the following. Additional definitions and explanations made further below also apply for all embodiments described in this specification mutatis mutandis.

The present invention further relates to a composition according to claim 9; further disclosure relates to a composition comprising a polynucleotide according to the present invention.

The term "composition", as used herein, as used herein, relates to a composition of matter comprising the compounds as specified and optionally one or more acceptable carrier. Preferably, the composition is a pharmaceutically acceptable composition; thus, preferably, the carrier is a pharmaceutically acceptable carrier. The compounds of the present invention can be formulated as, preferably pharmaceutically acceptable, salts. Preferred salts comprise acetate, methylester, HCl, sulfate, chloride and the like.

The carrier(s) must be acceptable in the sense of being compatible with the other ingredients of the formulation and being not deleterious to the recipient thereof. A carrier employed may be, for example, either a solid, a gel or a liquid. Exemplary of solid pharmaceutical carriers are lactose, terra alba, sucrose, talc, gelatin, agar, pectin, acacia, magnesium stearate, stearic acid and the like. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the compounds in the composition. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

Preferably, the composition mediates entry of the polynucleotide into a host cell. Thus, preferably the composition comprises at least one transfection agent. The selection of an appropriate transfection agent may depend on the target host cell, as well as the specific application envisaged. Transfection agents, appropriate transfection conditions, as well as selection criteria therefor are well-known in the art. Also preferably, the composition comprises virus-like particles. Thus, preferably, the polynucleotide is packaged into virus-like particles, i.e. preferably, the polynucleotide is comprised in the virus-like particles.

Pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well. For example, polynucleotide compounds may be administered in a gene therapy approach by using viral vectors or viruses or liposomes, as specified herein above. Moreover, the compounds can be administered in combination with other drugs either in a common pharmaceutical composition or as separated pharmaceutical compositions wherein said separated pharmaceutical compositions may be provided in form of a kit of parts. The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose of a pharmaceutical composition refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50.

The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. In case a viral vector, in particular adeno-associated viral vector is administered, preferred doses are from 5 × 1011, to 2 × 1013 viral particles or viral genomes / kg body weight; as will be understood, these exemplary doses may be modified depending, in addition to the factors described above, on additional factors like type of virus, target organ, and the like.

The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or users instructions in order to anticipate dose adjustments depending on the considered recipient.

The present invention also relates to a polynucleotide according to claim 1, a composition according to claim 9, and/or a host cell according to claim 10, for use in medicine. The present invention further relates to a polynucleotide according to claim 1, a composition according to claim 9, and/or a host cell according to claim 10, for use in treating genetic disease.

The term "genetic disease", as used herein, relates to a disease causally linked to one or more modifications, preferably mutations, in the genome of an individual. Thus, preferably, the genetic disease is causally linked to one or more epigenetic changes, more preferably is causally linked to one or more genetic mutations. As will be understood, symptoms of a genetic disease often are caused by expression of a mutated gene and/or lack of expression of a gene providing normal function of the gene product in one or more specific tissue(s) and/or cell type(s). Thus, it may be preferable to treat genetic disease only in those cells in which the mutation contributes to disease. Preferably, the genetic disease is a monogenic disease, i.e. is caused by a genetic alteration in one gene. More preferably, the genetic disease is a monogenic recessive disease, i.e. is caused by genetic alterations in both alleles of a gene; thus, preferably, the amelioration of symptoms is expected by provision of at least one unaltered copy of the affected gene. Most preferably, the genetic disease is phenylketonuria, alkaptonuria, Leber's Congenital Amaurosis, Choroideremia, or Stargardt disease. In a preferred embodiment, the genetic disease is cancer.

The present invention also relates to a kit comprising a polynucleotide according to claim 1 and a compound mediating cell entry.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to herein above. It is, preferably, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for interpreting the results obtained when carrying out the aforementioned methods using the kit of the present invention. As will be understood from the above, the description of the kit comprising polynucleotides, preferably, relates to a kit comprising corresponding vectors mutatis mutandis.

Preferably, the kit further comprises at least one compound mediating cell entry for the polynucleotide it comprises, the term "compound mediating cell entry" relating to any means suitable to cause a polynucleotide of the kit to enter the interior of a host cell, preferably a host cell. Suitable compound mediating cell entry (delivery means) are known in the art and include in particular transfection means, packaging compositions, and the like. Preferably, the polynucleotide of the present invention is pre-packaged in a delivery means, e.g. in viral particles, more preferably in replication-defective viral particles, most preferably in virus-like particles (VLPs). The skilled person is aware of delivery means providing different specificities for cellular receptors, such that delivery means appropriate for a given target host cell may be selected.

The present invention further relates to a device comprising a polynucleotide according to claim 1, a composition according to claim 9, and/or a host cell according to claim 10.

The term "device", as used herein relates to a system of means comprising at least the means operatively linked to each other as to allow administration of the compound or of the composition of the present invention. Preferred means for administering polynucleotides, compositions, host cells are well known in the art. How to link the means in an operating manner will depend on the type of means included into the device and on the kind of administration envisaged. Preferably, the means are comprised by a single device in such a case. Said device may accordingly include a delivery unit for the administration of the compound or composition and a storage unit for storing said compound or composition until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or composition of the invention. In another preferred embodiment, the device is an intravenous infusion (IV) equipment comprising the compound or composition. In another preferred embodiment, the device is an endoscopic device comprising the compound or medicament for flushing a site of administration, or further comprising a needle for topical application of the compound or composition, e.g. to a tumor. In still another preferred embodiment the device is an inhaler comprising the compound of the present invention, wherein, more preferably, said compound is formulated for administration as an aerosol.

The present application also relates to a method according to claim 14; also disclosed is a method for stably transfecting a host cell, comprising
a) contacting said host cell with a polynucleotide according to the present invention, a composition according to the present invention, and/or a host cell according to the present invention, and,
b) thereby, stably transfecting a host cell.

The method for stably transfecting a host cell of the present invention, preferably, is an in vitro method. Moreover, it may comprise steps in addition to those explicitly mentioned above. For example, further steps may relate, e.g., to providing a host cell or a sample comprising the same for step a), and/or applying selective pressure to the host cells contacted. Moreover, one or more of said steps may be performed by automated equipment.

The term "stably transfecting" a host cell is understood by the skilled person to relate to introducing a polynucleotide, preferably a heterologous polynucleotide into a cell such that the polynucleotide is stably replicated by the host cell as specified herein above. Preferably, stable transfection comprises stable episomal replication of the polynucleotide. Preferably, stable transfecting comprises, after contacting, applying selective pressure to the host cell to select for the presence of a selectable marker. The selective pressure is applied after contacting, optionally excluding a first time frame allowing the polynucleotide to establish within the host cell; the duration of said first time frame allowing the polynucleotide to establish within the host cell will depend mostly on the type of host cell contacted and on the kind of selectable marker used; preferably, the duration of said first time frame allowing the polynucleotide to establish within the host cell is of from 1 h to 48 h, more preferably of from 2 h to 24, most preferably of from 3 h to 16 h. However, the duration of said first time frame allowing the polynucleotide to establish within the host cell may also be zero, i.e. selective pressure may be applied immediately after contacting or even during contacting. Selective pressure may be applied continuously, i.e. at essentially all time points after the first time frame allowing the polynucleotide to establish within the host cell, more preferably to prevent host cells not comprising the polynucleotide from proliferating; or it may be applied transiently, more preferably to remove cells not having received the polynucleotide. Preferably, transient application of selective pressure is used in cases where cells are transferred back into an organism after said contacting. It is, however, also envisaged that no selective pressure is applied, in particular in cases where it is known that the efficiency of transfer of the polynucleotide into target host cells is sufficiently high and/or where a pure population of transgenic host cells is not of major importance. In a preferred embodiment, a stably transfected population of cells may also be obtained by allowing a cargo sequence encoding a detectable polypeptide to be expressed as specified herein above, and electing cells expressing the cargo sequence, e.g. by cell sorting, preferably FACS. In a preferred embodiment, stably transfecting comprises stable episomal replication of the polynucleotide, preferably episomal replication to such an extent that the polynucleotide still is detectable in a host cell population after on average 50 cell divisions.

The term "contacting", as used in the context of the methods of the present invention, is understood by the skilled person. Preferably, the term relates to bringing at least one polynucleotide, vector, and/or host cell of the present invention in physical contact with a host cell, e.g. allowing the host cell and the compound(s) to interact. Preferably, contacting includes delivery of at least one polynucleotide of the present invention into the interior of a host cell, preferably via a delivery means as specified above.

The present disclosure also relates to a method for treating genetic disease in a subject, comprising
a) contacting said subject with a polynucleotide according to the present invention, a composition according to the present invention, and/or a host cell according to the present invention, and,
b) thereby, treating genetic disease in said subject.

The method for treating genetic disease of the present disclosure, preferably, is an in vivo method. Moreover, it may comprise steps in addition to those explicitly mentioned above. For example, further steps may relate, e.g., to providing a host cell or a sample comprising the same for step a), and/or re-administering said sample or host cell into the subject. Thus, the method for treating genetic disease, comprise the steps of the method for stably transfecting a host cell as specified above. Moreover, one or more of said steps may be performed by automated equipment.

Further, the present invention relates to a use according to claim 15; also disclosed is a use of a polynucleotide of the present invention for stably genetically modifying a host cell.

Also, the present disclosure relates to a use of a polynucleotide according to the present invention, a composition according to the present invention, and/or a host cell according to the present invention, for the manufacture of a medicament. And to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease. In a preferred embodiment, the genetic disease is cancer, as specified herein above.

Also, the present disclosure relates to a use of a polynucleotide according to the present invention, a composition according to the present invention, and/or a host cell according to the present invention, for the genetic modification of a primary cell, preferably a primary dermal fibroblast, for the generation of an Induced Pluripotent Stem Cells (IPSCs). Preferably, said primary cell is a mouse or a human primary cell.

The term "primary cell" is understood by the skilled person as opposed to a cell of a cultured cell line; thus, preferably, a primary cell is a cell derived from a living organism and having been cultured for at most 20 passages, more preferably at most 15 passages, even more preferably at most 10 passages, still more preferably at most 5 passages. Most preferably, primary cells are cells derived directly from tissue of a living being, preferably a mouse or a human.

The term "stem cell" is also understood by the skilled person to relate to an un- or low-differentiated cell with the potential for differentiation into at least two cell types, preferably at least five cell types, more preferably at least one complete cell lineage. Preferably, the stem cell is a totipotent stem cell, more preferably a pluripotent stem cell. The term "Induced Pluripotent Stem Cell" or "IPSC" relates to a pluripotent stem cell derived from a differentiated cell, preferably a differentiated primary cell. Methods of generating IPSCs are known in the art and include, preferably, expression of four transcription factors in the cell (e.g from Takahashi et al. (2006), Cell. 126 (4):663).

The present disclosure also relates to a use of a polynucleotide according to the present invention, a composition according to the present invention, and/or a host cell according to the present invention, for the genetic modification of embryonic stem cells. The present disclosure also relates to a use of a polynucleotide according to the present invention, a composition according to the present invention, and/or a host cell according to the present invention, for the manufacture of a medicament for treating genetic disease, preferably monogenic disease, more preferably monogenic recessive disease, most preferably phenylketonuria, alkaptonuria, Leber's Congenital Amaurosis, Choroideremia, or Stargardt disease, wherein said medicament comprises host cells comprising a polynucleotide of the present invention.

The present disclosure also relates to a use of a polynucleotide according to the present invention, a composition according to the present invention, and/or a host cell according to the present invention, for the genetic modification of stem cells for generating a transgenic animal. The present disclosure further relates to a use of a polynucleotide according to the present invention, a composition according to the present invention, and/or a host cell according to the present invention, for the production of a transgenic animal.

The term "transgenic animal" as used herein, relates to an animal comprising at least one heterologous polynucleotide, preferably introduced into said animal by methods of genetic engineering. Preferably, the transgenic animal comprises at least one, more preferably at least 10, still more preferably at least 1000, even more preferably at least 10000 cells comprising at least one polynucleotide according to the present invention.

Also, the present disclosure relates to a use of a polynucleotide according to the present invention, a composition according to the present invention, and/or a host cell according to the present invention, for the genetic modification of single cell embryos by pronuclear injection.

As is understood by the skilled person, the term "pronuclear injection" relates to injecting genetic material, preferably a polynucleotide of the present invention, into the nucleus of a fertilized oocyte, preferably to create a transgenic animal.

The present disclosure further relates to the use of the polynucleotide according to the present invention or the composition according to the present invention in modifying gene expression in a host cell. To this end, polynucleotide sequences encoding interfering, non-coding nucleic acids may be included in expressible form. The non-coding interfering nucleic acid which is expressed from the polynucleotide, thus, may be typically an antisense RNA, siRNA, microRNA or ribozyme. Thereby, gene expression can be modified, i.e. down-regulated, by using the polynucleotide or composition according to the present invention. Expression vectors for interfering, non-coding nucleic acids comprising the polynucleotide of the invention have improved stability and expression performance but could simultaneously express the functional non-coding, interfering nucleic acids in a host cell or in a host organism. Therefore, the expression constructs comprising the polynucleotide of the invention and a polynucleotide in expressible form encoding a non-coding, interfering nucleic acid as specified above may also be used for gene silencing in a clinical context, i.e. for treating diseases or disorders including those mentioned in this specification elsewhere. Due to the improved stability and expression characteristics, gene silencing approaches can be improved as well.

### Figure Legends

Fig. 1: Efficiency of establishment and analysis of the genetically modified cell population: A) A cell culture plate with Crystal Violet stained colonies having formed after 4 weeks selection with Puromycin; the efficiency of vector establishment was approximately 40%; B) FACS detection of GFP fluorescence in Puromycin selected cells; fluorescence is very homogenous and the number of non-fluorescing cells is extremely low; 1= pEPI, 2= pSMARt.
Fig. 2: Result of plasmid rescue of pS/MARt vectors from established cell populations. Plasmid rescue and restriction analysis following bacterial transformation of total DNA derived from Hek293T cells established with pS/MARt and pEPI Plasmid DNA.
Fig. 3: Southern blot of pSMARt vectors maintained in selected cells: oligonucleotides hybridizing to the GFP gene of pS/MART were used as probes to detect BamHI restricted vector DNA in extracts from host cells (pS/MARt1 to 3); the non-transfected vector was used as a control ("pS/MARt(+)").
Fig. 4: Vector map of pS/MART; ori: bacterial origin of replication, P2A: sequence encoding the self-cleaving 2A peptide from porcine teschovirus-1, apolipoB MAR: S/MAR sequence from the apolipoprotein B gene.
Fig. 5: Colony forming assay with different versions of pSMARt in Hek293T and HeLa cells
Fig. 6: pSMARt is efficiently retained in dividing cells also in the absence of continuous selection
Fig. 7: The efficiency of establishment of pSMARt is independent of the selection marker (A), but the presence of an insulator element before the promoter increases its establishment efficiency (B); Fig 7 A : 1=pEPI, 2 pSMARt-Ele40-GFP-2A-Puro, 3=pSMARt-Ele40-GFP-2A-G418; B: 1=pSMARt-UCOE, 2=pSMARt-Ele40, 3=pSMARt, 4=pEPI
Fig. 8: pSMARt vectors are efficiently retained in primary human CD3+ cells for over a month.
Fig. 9: The introduction of splice junctions flanking the SMAR improves the establishment of the vectors in dividing cells. (1) pEPI, (2) pS/MARt, (3) NP, (4) NP-SPlice (1,2,3,4 all with β-inf MAR) (5) pS/MARt.
Fig. 10: pSMARt is maintained in mouse embryonic stem cells (mESC) and the presence of the vector does not influence the cells' behavior.
Fig. 11: pSMARt vectors are active during embryogenesis and differentiation, and they are resistant to epigenetic silencing
Fig. 12: pSMARt sustains the expression of the transgene GFP during stem-cell differentiation
Fig. 13: A comparison of CAR-T cells modified with pSMARt vectors and lentivirus; Real Time tumor killing, interferon production and cytotoxicity analysis.
Fig. 14: Analysis of tumor killing in vivo
Fig. 15: Analysis of tumor samples and maintenance in vivo of CAR T cells modified with pSMARt and lentivirus; 1= Isotype control, 2= untreated, 3= treated with mock T cells, 4= T cells transduced with lentivirus encoding for the CAR anti human CEA, 5= T cells transfected with pSMARt encodigin for the CAR anti human CEA

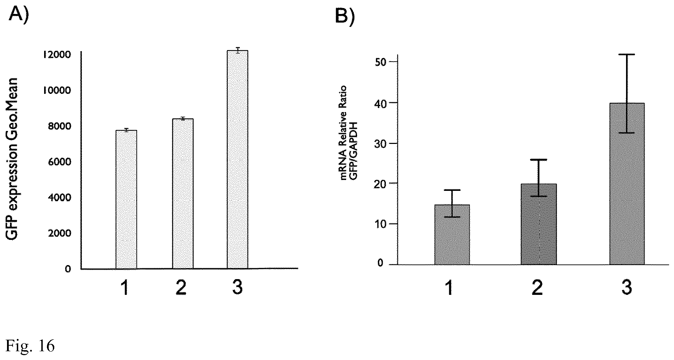

Fig. 16: Expression data from pS/MARt and Nano vectors containing the splicing sequences. Hek293T cell populations established with different version of pS/MARt were analyzed for transgene expression 35 days post DNA delivery and selection in Puromycin (0.5 ug/ml) by Flow Cytometry (A). The relative expression of the transgene GFP was evaluated and normalized to the expression of the housekeeping gene GAPDH (B). The figure shows that the introduction of splicing sequences improve the transgene expression by improving the RNA amount in the cells. Figure (A): (1) pS/MARt, (2) Nano-S/MARt, (3) Nano-S/MARt-splice; Figure (B): (1) pS/MARt, (2) Nano-S/MARt, (3) Nano-S/MARt-splice.

The following Examples shall merely illustrate the invention. They shall not be construed, whatsoever, to limit the scope of the invention.

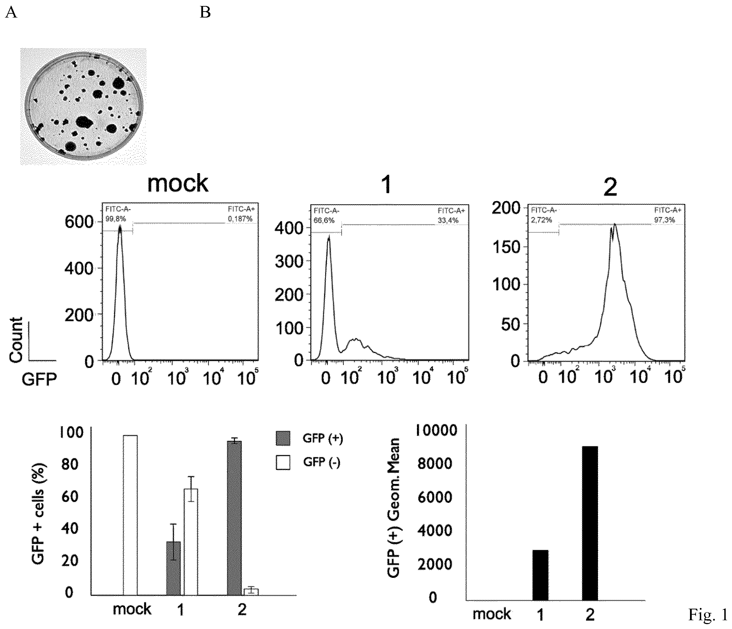

### Example 1: Efficiency of establishment and analysis of the genetically modified cell population (Fig. 1)

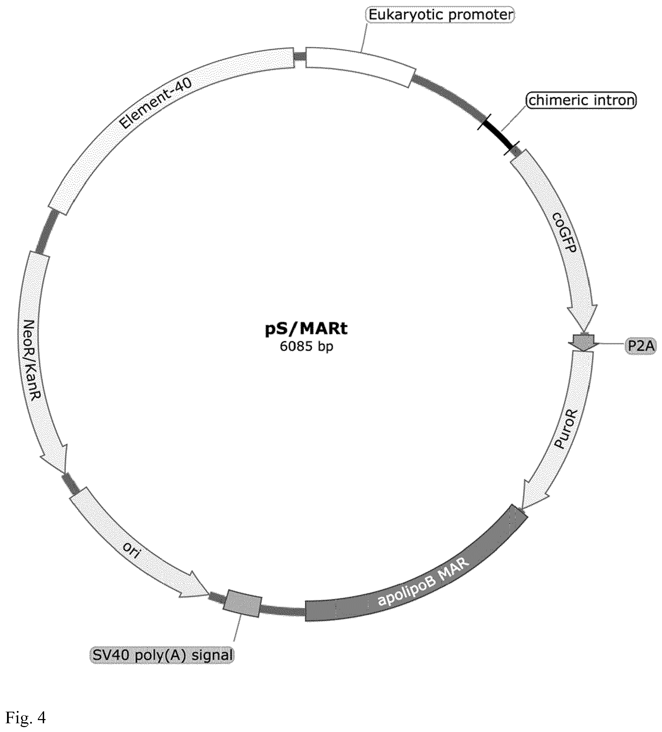

The efficacy in generating stably expressing cells was evaluated in a colony forming assay using pS/MARt compared to pEPI (Fig. 4, SEQ ID NO:14). Upon DNA delivery, cells positive for GFP transgene expression were isolated via FACS sorting (FACS Aria II) and 100 cells were plated into a 6 cm cell culture dish. They were then cultured for 4 weeks in presence of 0.5 µg/ml Puromycin. After 4 weeks the cells were fixed with PFA and the colonies stained with Crystal Violet. The number of colonies is considered as the efficiency of vector establishment, i.e. the number of colonies forming per number of FACS sorted cells plated. The generation of stable cells lines is very effective with over 40 % of transfected cells becoming established (Fig. 1A)). The number of transgene (GFP) expressing cells was estimated by Flow Cytometry. As shown in Fig. 1 b), pS/MARt generates modified populations in which the expression of the transgene is homogenous without significant numbers of negative cells.

The efficienncy of generating stably expressing cells was evaluated by a Flow Cytometry. Hek293T cells were transfected with either pEPI or pS/MARt. The cells were cultured for 35 days and then subjected to FACS analysis to determine the proportion of cells which continued to express the transgene(GFP) and their medium fluorescent intensity. The upper panels of Fig 1 B) show that pS/MARt generates a population of genetically modified cells in which the expression of the transgene is robust and homogenous. This is in contrast to cells transfected with pEPI which show heterogeneous and low levels of transgene expression. The bar charts show that in that in these cellular pools there is no significant population of negative cells in the pS/MARt whilst the pEPI lines have predominantly silenced or lost the transgene expression. Additionally, in the pS/MARt lines median expression of the transgene is substantially higher. Experimental details: cell transfection with JetPEI, selection with 0.5 ug/ml Puro and 1mg/ml G418 for pEPI.

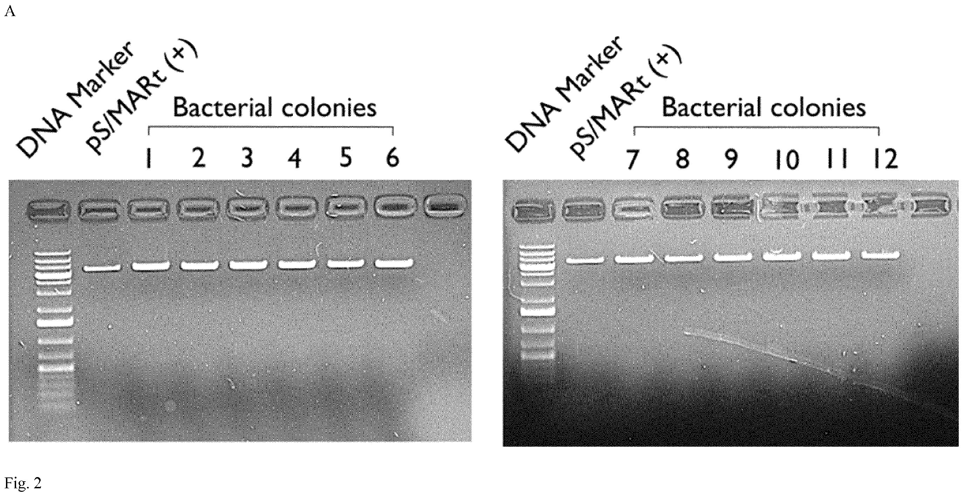

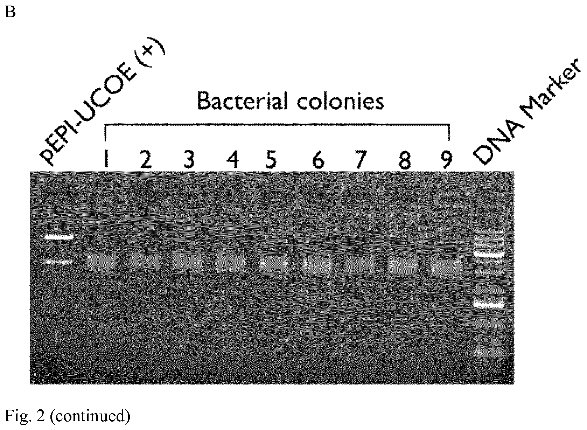

### Example 2: Plasmid rescue of pS/MARt vectors from established cell populations (Fig. 2)

We performed DNA rescue experiments to determine the episomal status and the molecular integrity of the vector in mammalian cells.

Vector DNA was isolated from cells either established with the plasmid pS/MARt or pEPI. These cells were expanded in the presence of the antibiotic Puromycin (0.5 ug/ml) for 1 week and further expanded for at least 30 days without antibiotic. For the plasmid rescue the gDNA from established cells was extracted with the Blood&Tissue DNAeasy kit (Qiagen) and transformed into DH10B E.Coli. Bacteria were grown on LB-Agar plates with Kanamycin (50 ug/ml). 12 colonies were grown in liquid LB medium with Kanamycin (50 ug/ml) over night and plasmid DNA was extracted with the MiniprepKit (Qiagen). For the analysis the DNA mini preparations were digested with the restriction enzyme BamHI (Thermo Fisher) for 10 min at 37°C and the restriction pattern was addressed on a 1% agarose gel. As control the DNA used for transfecting the cells at the beginning of the establishment procedure was digested with the same enzyme and run as a reference. (A) 12 representative samples isolated from pS/MART were analysed and proved to be molecularly equivalent to the original DNA Vector whilst DNA rescued from cells established with pEPI (B) was shown to be molecularly dissimilar to the original vector with smeared bands clearly showing rearrangements of the original DNA.

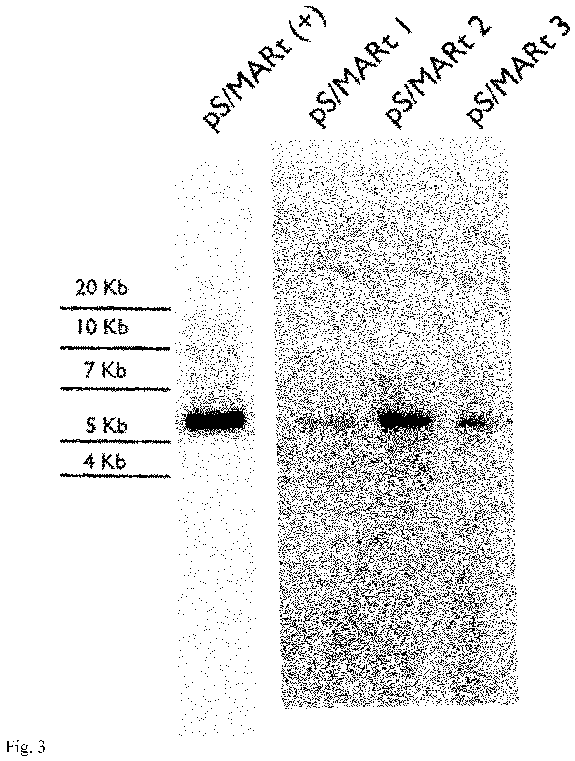

### Example 3: pS/MARt vectors are maintained episomally in modified cells (Fig. 3)

To further demonstrate that the pS/MARt vectors were modifying the mammalian cells as an episome, structure was physically determined by Southern Blot analysis. Hek293T cell populations cultured for at least 30 days after DNA transfection were analyzed. The genomic DNA was extracted with the Blood&Tissue DNAeasy kit (Qiagen) and digested over night at 37°C with the restriction enzyme BamHI (NEB). The total cellular DNA was then separated on a 1% agarose gel and transferred to a nylon membrane. Oligonucleotides corresponding to the vector's GFP gene were used to generate the radioactive probe used to detected the pS/MARt DNA within cellular DNA. The presence in the samples of a single band that has the same size of the control vector demonstrates the episomal status of pS/MARt in the established mammalian cell populations. The absence of smears and/or alternative bands demonstrates that the vectors did not rearrange nor integrate into the cellular genome.

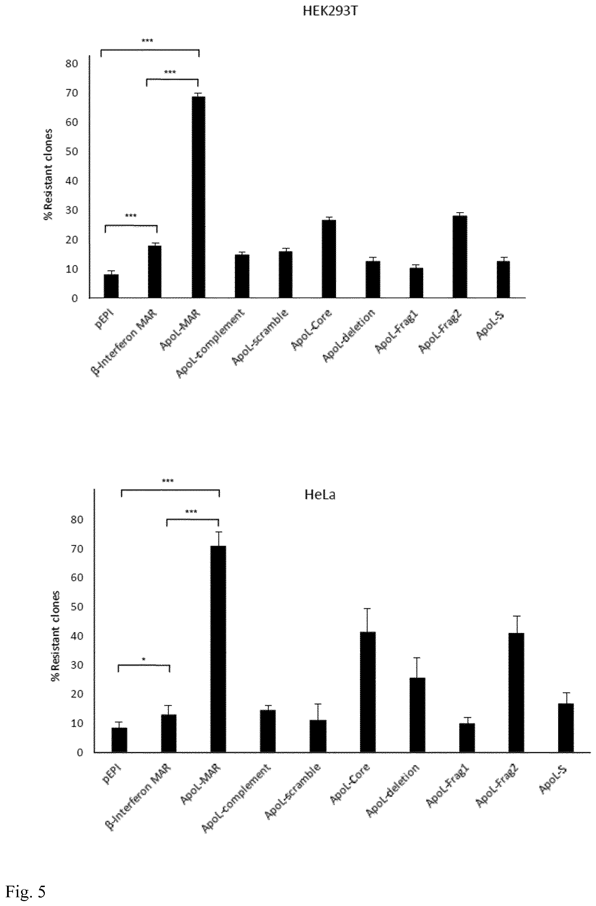

### Example 3: Efficiency in generating stably expressing cells (Fig. 5)

The efficiency in generating stably expressing cells by a range of pS/MARt DNA Vectors harbouring components of the ApoL-MAR and the beta-IFN-MAR was evaluated in a colony forming assay. Upon DNA delivery, cells positive for the expression of the reporter gene GFP were isolated via FACS sorting (FACS Aria II) and 100 were plated into a 6 cm cell culture dish. The cells were then cultured for 4 weeks in the presence of 0.5 g/ml Puromycin. After 4 weeks the cells were fixed with PFA and the colonies stained with Crystal Violet and quantified. The number of colonies is considered as the vector establishment efficiency .The assay shows that vectors engineered with the ApoL MAR, the Core sequence or Fragment 2 are the most efficient in the generation of genetically modified cells.

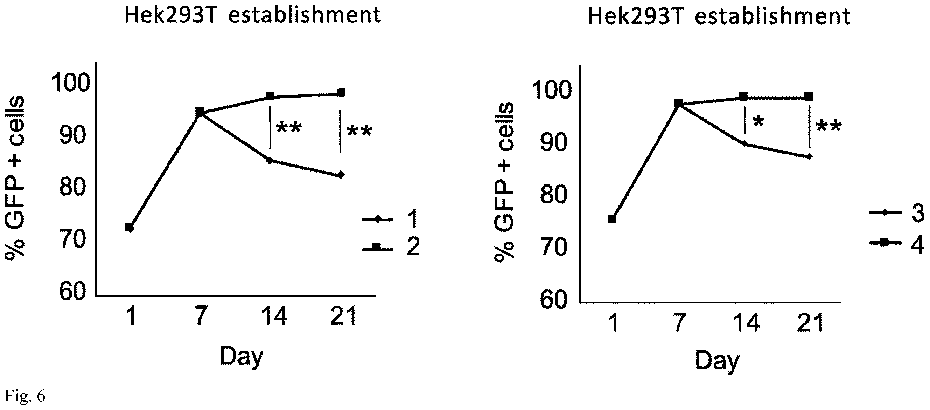

### Example 4: Stability in the absence of selection (Fig. 6)

To evaluate whether the insulator sequence could prevent the silencing or loss of DNA vectors we measured the transgene expression of GFP in cells transfected with either (A) pSMARt-insulator-GFP-2a-Puro beta interferon MAR or (B) pSMARt-insulator-GFP-2a-Puro ApoL MAR in the absence of antibiotic selection. Cells were transfected with DNA vectors and cultured in 1 ug/ml Puromycin. After one week the cells were split and the drug was removed form a representative group of cells from each transfection.
(1) pSMARt-insulator-GFP-2a-Puro beta interferon MAR - cultured in continuous selection
(2) pSMARt-insulator GFP-2A-Puro beta interferon MAR - selection removed
(3) pSMARt-insulator-GFP-2a-Puro ApoL MAR - cultured in continuous selection
(4) pSMARt-insulator GFP-2A-Puro ApoL MAR - selection removed

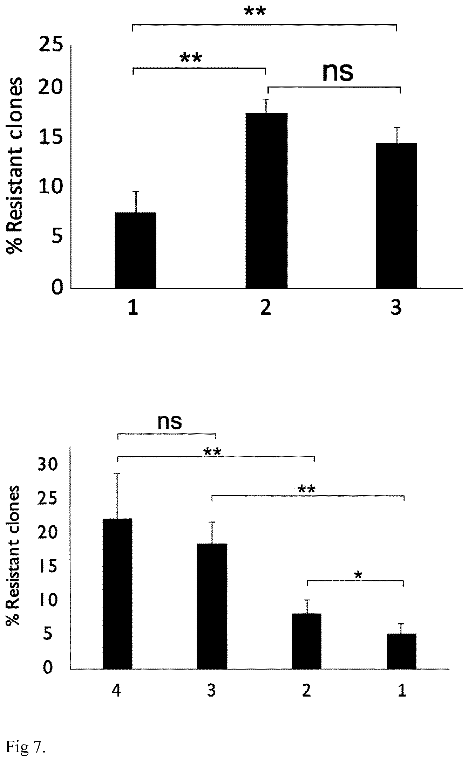

### Example 5: Establishment efficiency (Fig. 7)

To evaluate the influence of the selection marker on the establishment efficiency we performed colony forming assays as described in Figure 5. Figure 7 (A) shows that the establishment efficiency with the DNA vector pS/MARt is independent of the antibiotic selection marker. The selection marker improves establishment efficiency when it is transcriptionally linked to the S/MAR motif. Figure 7 (B) illustrates improved efficiency of establishment with the additional incorporation of an insulating element between the bacterial backbone and the eukaryotic promoter.

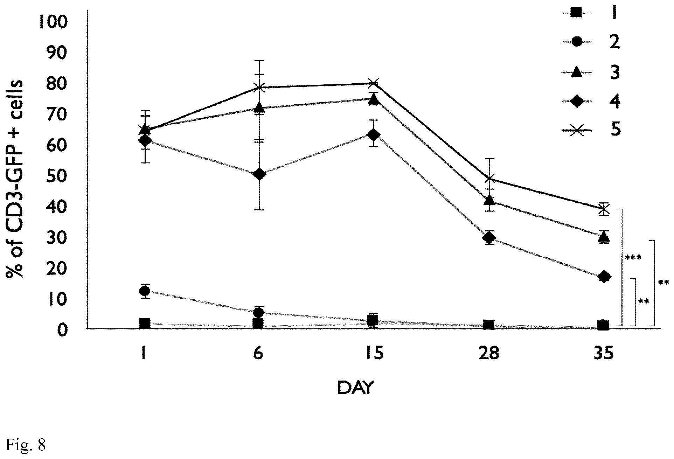

### Example 6: Stability in primary cells (Fig. 8)

To evaluate the efficacy of our DNA vector system to transfect primary human CD3+ cells and to measure their expression profiles we transfected three variants of the pS/MARt vector system and pEPI encoding GFP and tested their capability for providing sustained expression of the reporter gene GFP in human T cells. The DNA Vectors were delivered to freshly isolated PBMCs via electro- transfer (Nucleofector Device Y, Lonza) and the cells were cultured in presence of IL-2 (5µg/ml, Biolegend) for 35 days . Every 7 days the cells were checked for the transgene expression and their growth was stimulated via addition in the media of the antibody anti-CD28(Bioegend) and anti-CD3 (Biolegend). pS/MARt carrying the core version of the ApoLMAR is able to generate a higher number of transgene expressing cells gene when compared to the full length ApoL-MAR sequence, to pS/MARt harbouring the βIPN MAR and to pEPI.

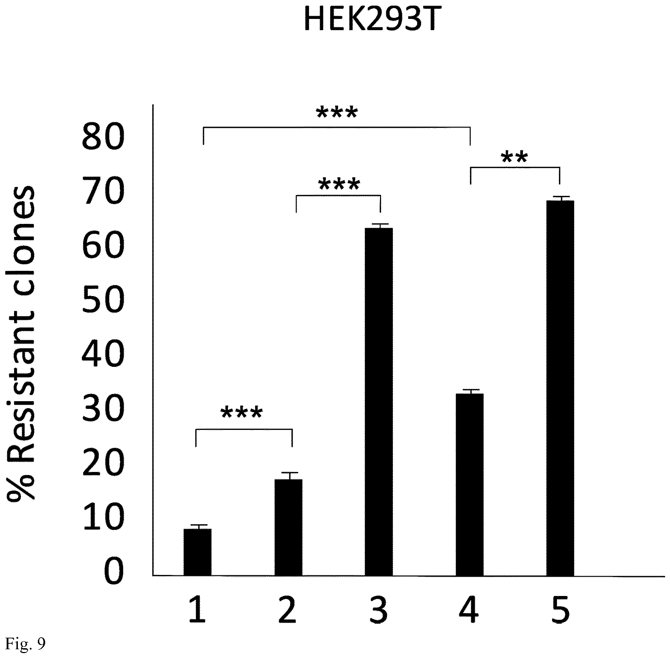

### Example 7: MAR splicing (Fig. 9)

To demonstrate that the efficiency of establishment could be improved by the introduction of splicing sequences we generated a series of DNA Vectors with and without the splice donor and acceptor sites flanking the MAR element. To evaluate the influence of the splicing elements on the establishment efficiency we performed colony forming assays as described in Figure 5.

The efficiency of establishment of the DNA vectors are significantly improved upon the improvement and minimalization of the bacterial backbone. With the improvements listed previously namely the selction marker and the insulater sequences and its more modern and improved backbone pS/MARt establishes more efficiently than pEPI. In turn minimalization of the backbone Figure 9 (3) improves this further. The introduction of splicing sequences to remove the S/MAR element from the mRNA nearly doubles the efficiency of this class of vector. This effect can be reproduced with similar compositions of vectors comprising different S/MAR elements (5-6).

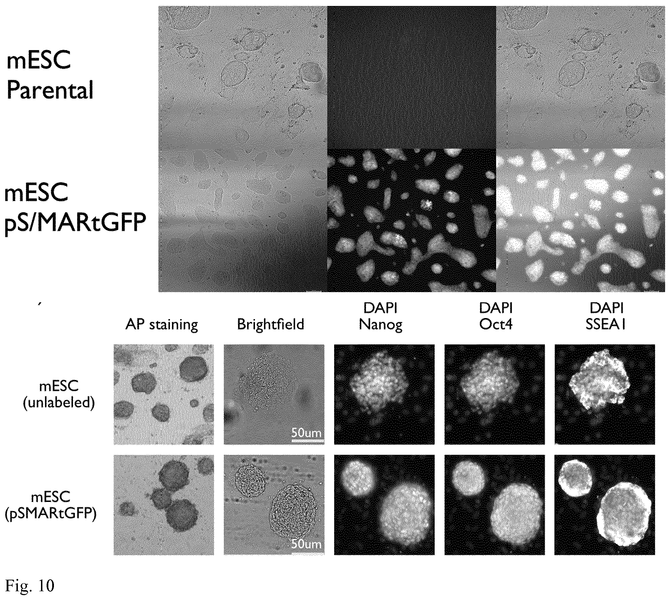

### Example 8: pS/MARt in stem cells (Fig. 10)

To evaluate whether pS/MARt could effectively modify stem cells without molecular damage we generated stable stem cell lines. The E14 mouse embryonic stem cell (mESC) line was established with the pS/MARt-GFP vector. The expression of the reporter gene GFP was measured 1 month after DNA delivery via fluorescent microscopy (A). mESC modfied with pS/MARt-GFP were stained for the most common pluripotency markers showing that the presence of the episomal vector does not alter their pluripotent features.

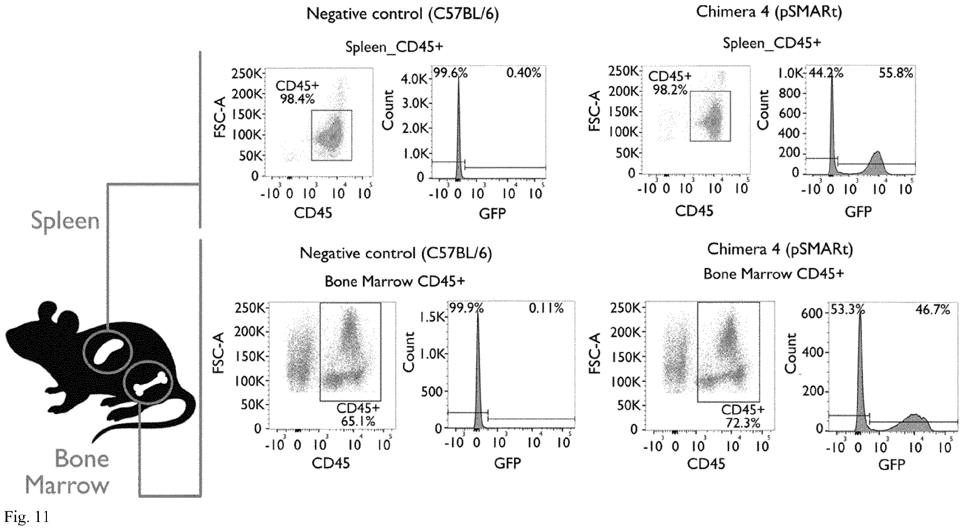

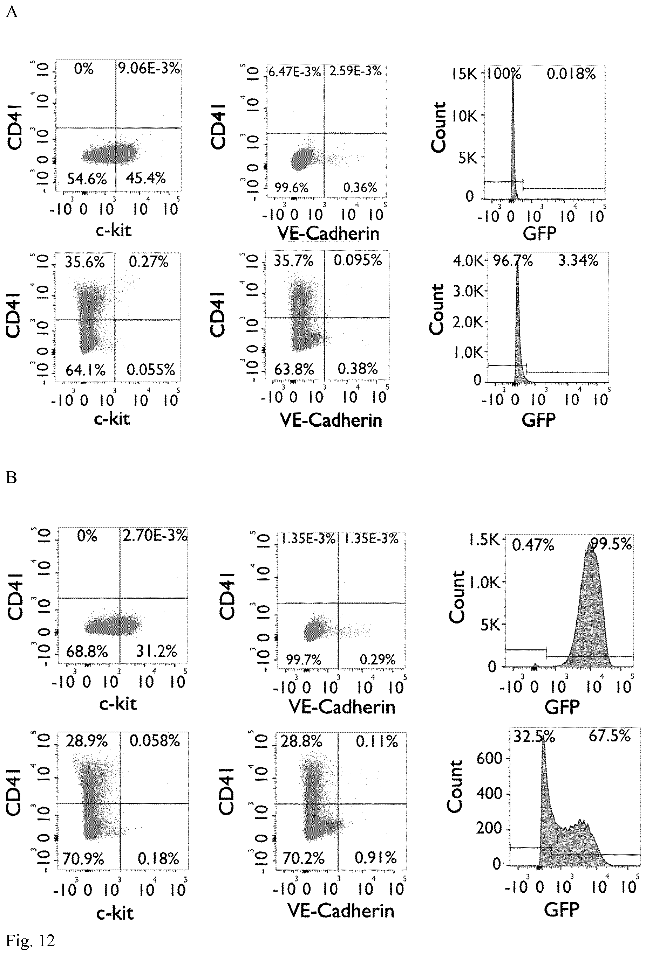

### Example 9: pS/MARt in embryogenesis (Fig. 11 and 12)

pSMARt-GFP labeled mESC were injected into blastocysts of C57BL/6 mice, which results in the formation of chimeras. Hematopoietic organs from these chimeras, such as spleen and bone marrow, were stained with a pan blood surface marker (CD45) and their fluorescence analyzed by flow cytometry. A C57BL/6 mouse and a constitutively expressing UBC::GFP mouse, were used as negative and positive controls respectively. (Fig. 11)

pS/MARt mediates the persistent expression of a transgene during hematopoietic differentiation. mESC were forced to differentiate into HSC and they were analysed by Flow Cytometry before (day 0) and afer (day 6) the differentiation process. Both, parental and labelled cells were successfully differentiated into HSC and cells tagged with pS/MARt-GFP retained the expression of the reporter gene throughout the procedure. (Fig. 12)

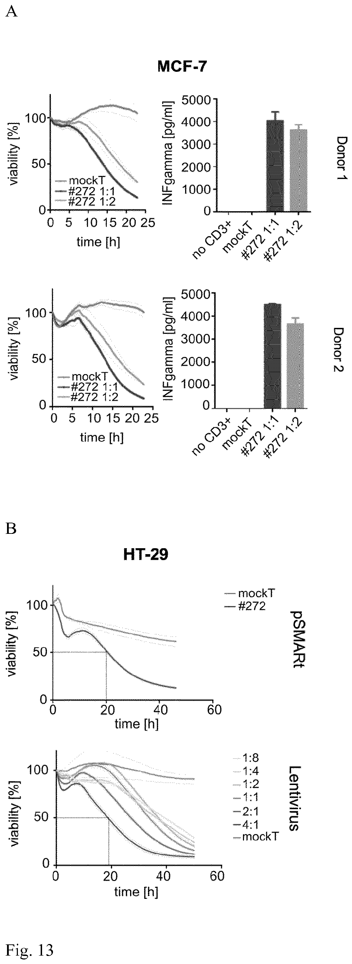

### Example 10: comparison with lentivirus (Fig. 13)

(A) CD3+ cells sorted from two different healthy donors were modified with S/MARt DNA Vectors expressing the CAR272 which provides targeting against the human CEA epitope and their cytolytic activity was confirmed in MCF-7 cells (a breast cancer cell line) with cytotoxic and Interferon-y releasing assays.
(B) Engineered T-cells with the S/MARt Vector system expressing the CAR272 shows an improved killing activity when compared to CD3+ cells modified with lentiviruses carrying the same expression cassette.

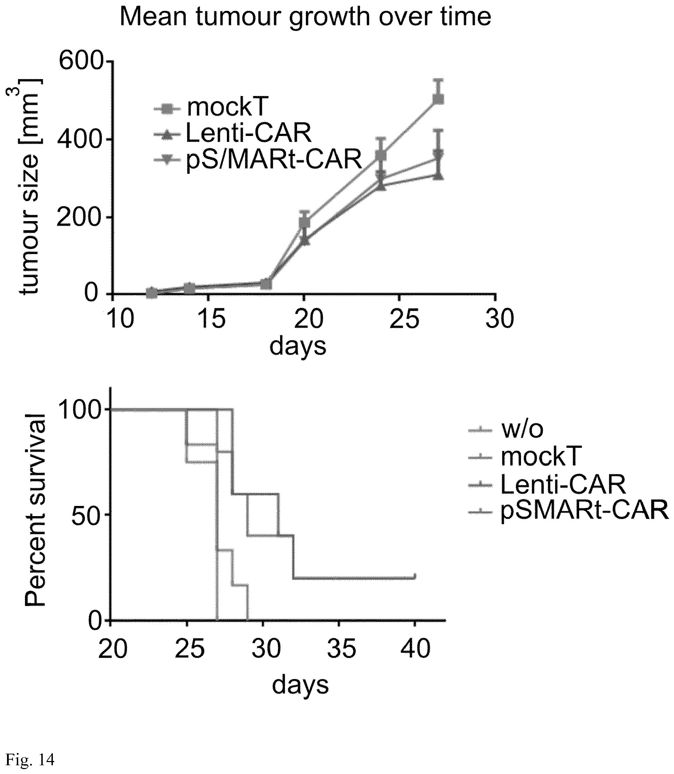

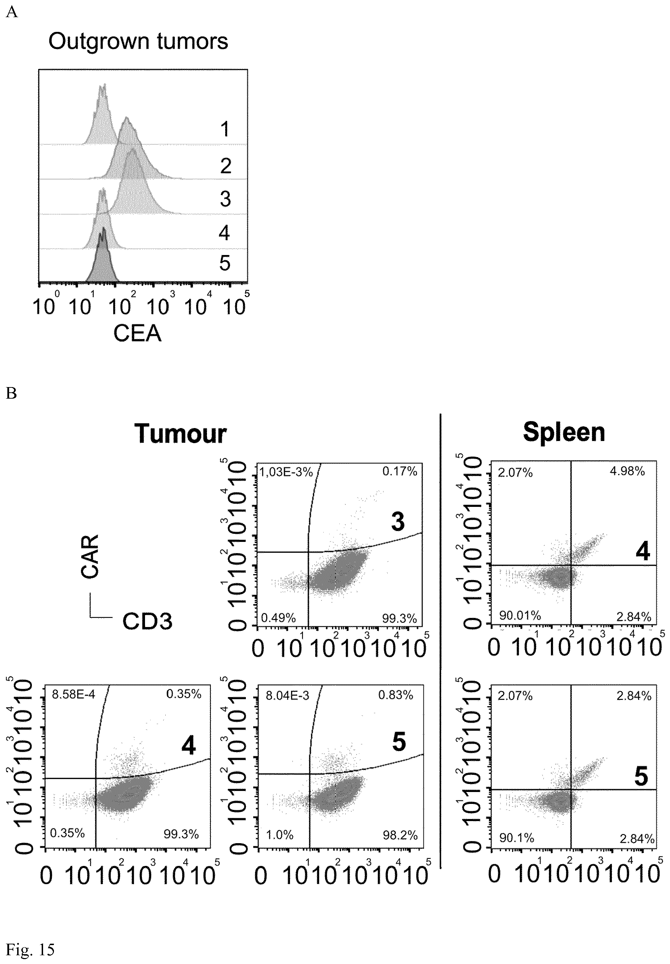

### Example 11: Tumor killing (Fig. 14 and 15)

In vivo analysis of CAR-T cells produced with the S/MARt DNA Vector system, lentivirus and the next-generation NanoS/MARt DNA Vector. (Fig. 14)

NOD/SCID mice (n=6) were inoculated subcutaneously with 2×10⁶ HT29 tumor cells. 3×10⁵ CAR+ T-cells generated with the S/MARt DNA Vector, lentivirus and the next-generation were injected into the tail vein of each mouse without prior chemo- or radiotherapy at day 7 post tumour cells injection. The tumour targeting efficacy of the modified cells was compared to mock electroporated CD3+ and recorded as tumour growth (A) and mice survival (B).

The outgrown tumours (isolated from the tumours described above) were explanted, dissociated and analysed for the presence of the CAR target (Fig. 15). The frequency of CAR+ CD3 within tumor masses was analaysed as well as the presence of CAR expressing CD3 in the spleen.

The remaing tumours comprise only tumour cells that lack the targeted epitope illustrating that the S/MARt treated T-Cells were effective at killing and clearing tumour cells at at least comparable levels to the lentiviral control. Additionally CAR-T positive cells are still detectable infiltrating within the tumour and populating the spleen indicating that the DNA vector is still actively expressing within the transgenic T-Cells.

## Claims

1. A polynucleotide comprising at least one promoter and an S/MAR element, wherein said S/MAR element is located downstream of said promoter and wherein the nucleic acid sequence of said S/MAR element (S/MAR sequence) comprises at least 3 sequence motifs ATTA (SEQ ID NO:1) per 100 nucleotides over a stretch of at most 200 nucleotides, wherein said S/MAR element is flanked by a splice donor and a splice acceptor.

2. The polynucleotide of claim 1, wherein said polynucleotide further comprises a coding sequence encoding a polypeptide, wherein said coding sequence encoding a polypeptide intervenes said promoter and said S/MAR element.

3. The polynucleotide of claim 1 or 2, wherein said polynucleotide further comprises a coding sequence encoding a selectable marker (selectable marker sequence), said selectable marker sequence intervening said promoter and said S/MAR element, wherein said promoter and said selectable marker sequence together constitute a selectable marker gene, and wherein said selectable marker is a selectable marker of a eukaryotic cell.

4. The polynucleotide of claim 3, wherein said selectable marker gene is a puromycin resistance gene, a blasticidin resistance gene, a neomycin resistance gene, or a zeocin resistance gene.

5. The polynucleotide of claim 3 or 4, wherein said selectable marker gene is a puromycin resistance gene.

6. The polynucleotide of any one of claims 1 to 5, wherein a transcript is transcribed from said promoter, from which transcript the sequence of the S/MAR element is spliced out.

7. The polynucleotide of any one of claims 1 to 6, wherein said polynucleotide is devoid of a simian virus 40 (SV40) origin of replication, a bovine papillomavirus (BPV) origin of replication, and an Epstein-Barr virus (EBV) origin of replication.

8. The polynucleotide of any one of claims 1 to 7, wherein said polynucleotide replicates episomally in a host cell, preferably wherein episomal replication is stable episomal replication, preferably in a mammalian cell

9. A composition comprising a polynucleotide according to any one of claims 1 to 8, preferably wherein said composition is a pharmaceutical composition.

10. A host cell comprising the polynucleotide according to any one of claims 1 to 8, preferably wherein said host cell is a CD34+ Progenitor Cell; a CD61+ Thrombocyte; a CD19+ B-Lymphocyte; a CD14+ Monocyte; a CD15+ Granulocyte; a CD3+ Cytotoxic T-Lymphocyte, preferably also positive for CD8 and CD45; a CD3+ Helper T-Lymphocyte, preferably also positive for CD4 and CD45; a CD3+ activated T-Lymphocyte, preferably also positive for CD25 and CD45, a Tumor infiltrating Lymphocyte, or a Natural Killer (NK) cell.

11. A polynucleotide according to any one of claims 1 to 8, a composition according to claim 9, and/or a host cell according to claim 10, for use in medicine, preferably, for use in treating genetic disease.

12. A kit comprising a polynucleotide according to any one of claims 1 to 8 and a compound mediating cell entry.

13. A device comprising a polynucleotide according to any one of claims 1 to 8, a composition according to claim 9, and/or a host cell according to claim 10.

14. A method for stably transfecting a host cell, comprising
a) contacting said host cell with a polynucleotide according to any one of claims 1 to 8, and/or a composition according to claim 9, and/or a host cell according to claim 10, and,
b) thereby, stably transfecting a host cell.

15. Use of a polynucleotide according to any one of claims 1 to 8 for stably genetically modifying a host cell.

## Patentansprüche

1. Polynukleotid, umfassend mindestens einen Promotor und ein S/MAR-Element, wobei sich das S/MAR-Element stromabwärts des Promotors befindet und wobei die Nukleinsäuresequenz des S/MAR-Elements (S/MAR-Sequenz) mindestens 3 Sequenzmotive ATTA (SEQ ID NO:1) pro 100 Nukleotide über eine Abfolge von höchstens 200 Nukleotiden umfasst, wobei das S/MAR-Element von einem Spleißdonor und einem Spleißakzeptor flankiert ist.

2. Polynukleotid nach Anspruch 1, wobei das Polynukleotid ferner eine codierende Sequenz umfasst, die ein Polypeptid codiert, wobei die ein Polypeptid codierende Sequenz zwischen dem Promotor und dem S/MAR-Element liegt.

3. Polynukleotid nach Anspruch 1 oder 2, wobei das Polynukleotid ferner eine codierende Sequenz umfasst, die einen Selektionsmarker codiert (Selektionsmarkersequenz), wobei die Selektionsmarkersequenz zwischen dem Promotor und dem S/MAR-Element liegt, wobei der Promotor und die Selektionsmarkersequenz zusammen ein Selektionsmarkergen bilden und wobei es sich bei dem Selektionsmarker um einen Selektionsmarker einer eukaryotischen Zelle handelt.

4. Polynukleotid nach Anspruch 3, wobei das Selektionsmarkergen ein Puromycinresistenzgen, ein Blasticidinresistenzgen, ein Neomycinresistenzgen oder ein Zeocinresistenzgen ist.

5. Polynukleotid nach Anspruch 3 oder 4, wobei das Selektionsmarkergen ein Puromycinresistenzgen ist.

6. Polynukleotid nach einem der Ansprüche 1 bis 5, wobei ein Transkript von dem Promotor transkribiert wird, wobei aus dem Transkript die Sequenz des S/MAR-Elements herausgespleißt wird.

7. Polynukleotid nach einem der Ansprüche 1 bis 6, wobei das Polynukleotid keinen Simian-Virus 40(SV40)-Replikationsursprung, keinen Bovines-Papillomvirus(BPV)-Replikationsursprung und keinen Epstein-Barr-Virus(EBV)-Replikationsursprung aufweist.

8. Polynukleotid nach einem der Ansprüche 1 bis 7, wobei das Polynukleotid episomal in einer Wirtszelle repliziert, wobei es sich vorzugsweise bei der episomalen Replikation um stabile episomale Replikation, vorzugsweise in einer Säugerzelle, handelt.

9. Zusammensetzung, umfassend ein Polynukleotid nach einem der Ansprüche 1 bis 8, wobei die Zusammensetzung vorzugsweise eine pharmazeutische Zusammensetzung ist.

10. Wirtszelle, umfassend das Polynukleotid nach einem der Ansprüche 1 bis 8, wobei die Wirtszelle vorzugsweise eine CD34+ Vorläuferzelle; ein CD61+ Thrombozyt; ein CD19+ B-Lymphozyt; ein CD14+ Monozyt; ein CD15+ Granulozyt; ein zytotoxischer CD3+ T-Lymphozyt, der vorzugsweise auch positiv für CD8 und CD45 ist; ein CD3+ Helfer-T-Lymphozyt, der vorzugsweise auch positiv für CD4 und CD45 ist; ein aktivierter CD3+ T-Lymphozyt, der vorzugsweise auch positiv für CD25 und CD45 ist, ein tumorinfiltrierender Lymphozyt oder eine natürliche Killer(NK)-Zelle ist.

11. Polynukleotid nach einem der Ansprüche 1 bis 8, Zusammensetzung nach Anspruch 9 und/oder Wirtszelle nach Anspruch 10 zur Verwendung in der Medizin, vorzugsweise zur Verwendung bei der Behandlung einer genetischen Krankheit.

12. Kit, umfassend ein Polynukleotid nach einem der Ansprüche 1 bis 8 und eine den Zelleintritt vermittelnde Verbindung.

13. Vorrichtung, umfassend ein Polynukleotid nach einem der Ansprüche 1 bis 8, eine Zusammensetzung nach Anspruch 9 und/oder eine Wirtszelle nach Anspruch 10.

14. Verfahren zum stabilen Transfizieren einer Wirtszelle, umfassend
a) Inkontaktbringen der Wirtszelle mit einem Polynukleotid nach einem der Ansprüche 1 bis 8 und/oder einer Zusammensetzung nach Anspruch 9 und/oder einer Wirtszelle nach Anspruch 10 und
b) dadurch stabiles Transfizieren einer Wirtszelle.

15. Verwendung eines Polynukleotids nach einem der Ansprüche 1 bis 8 zum stabilen genetischen Modifizieren einer Wirtszelle.

## Revendications

1. Polynucléotide comprenant au moins un promoteur et un élément S/MAR, ledit élément S/MAR étant situé en aval dudit promoteur et la séquence d'acides nucléiques dudit élément S/MAR (séquence S/MAR) comprenant au moins 3 motifs de séquence ATTA (SEQ ID NO: 1) par 100 nucléotides sur une longueur d'au plus 200 nucléotides, ledit élément S/MAR étant flanqué d'un donneur d'épissage et d'un accepteur d'épissage.

2. Polynucléotide selon la revendication 1, ledit polynucléotide comprenant en outre une séquence codante codant pour un polypeptide, ladite séquence codante codant pour un polypeptide étant intercalée entre ledit promoteur et ledit élément S/MAR.

3. Polynucléotide selon la revendication 1 ou 2, ledit polynucléotide comprenant en outre une séquence codante codant pour un marqueur sélectionnable (séquence de marqueur sélectionnable), ladite séquence de marqueur sélectionnable étant intercalée entre ledit promoteur et ledit élément S/MAR, ledit promoteur et ladite séquence de marqueur sélectionnable constituant ensemble un gène de marqueur sélectionnable, et ledit marqueur sélectionnable étant un marqueur sélectionnable d'une cellule eucaryote.

4. Polynucléotide selon la revendication 3, ledit gène de marqueur sélectionnable étant un gène de résistance à la puromycine, un gène de résistance à la blasticidine, un gène de résistance à la néomycine, ou un gène de résistance à la zéocine.

5. Polynucléotide selon la revendication 3 ou 4, ledit gène de marqueur sélectionnable étant un gène de résistance à la puromycine.

6. Polynucléotide selon l'une quelconque des revendications 1 à 5, un transcrit étant transcrit dudit promoteur, à partir duquel un transcrit de la séquence de l'élément S/MAR est épissé.

7. Polynucléotide selon l'une quelconque des revendications 1 à 6, ledit polynucléotide étant dépourvu d'une origine de réplication du virus simien 40 (SV 40), d'une origine de réplication du papillomavirus bovin (BPV) et d'une origine de réplication du virus d'Epstein-Barr (EBV).

8. Polynucléotide selon l'une quelconque des revendications 1 à 7, ledit polynucléotide se répliquant de manière épisomale dans une cellule hôte, préférablement la réplication épisomale étant une réplication épisomale stable, préférablement dans une cellule de mammifère.

9. Composition comprenant un polynucléotide selon l'une quelconque des revendications 1 à 8, préférablement, ladite composition étant une composition pharmaceutique.

10. Cellule hôte comprenant le polynucléotide selon l'une quelconque des revendications 1 à 8, préférablement, ladite cellule hôte étant une cellule progénitrice CD34+ ; un thrombocyte CD61+ ; un lymphocyte B CD19+ ; un monocyte CD14+ ; un granulocyte CD15+ ; un lymphocyte T cytotoxique CD3+ ; préférablement également positif pour CD8 et CD45 ; un T-Lymphocyte auxiliaire CD3+, préférablement également positif pour CD4 et CD45 ; un T-Lymphocyte activé CD3+, préférablement également positif pour CD25 et CD45, un lymphocyte infiltrant les tumeurs, ou une cellule Natural Killer (NK).

11. Polynucléotide selon l'une quelconque des revendications 1 à 8, composition selon la revendication 9, et/ou cellule hôte selon la revendication 10, pour une utilisation en médecine, préférablement, pour une utilisation dans le traitement d'une maladie génétique.

12. Kit comprenant un polynucléotide selon l'une quelconque des revendications 1 à 8 et un composé médiateur de l'entrée dans les cellules.

13. Dispositif comprenant un polynucléotide selon l'une quelconque des revendications 1 à 8, une composition selon la revendication 9, et/ou une cellule hôte selon la revendication 10.

14. Procédé pour la transfection de manière stable d'une cellule hôte, comprenant
a) la mise en contact de ladite cellule hôte avec un polynucléotide selon l'une quelconque des revendications 1 à 8, et/ou une composition selon la revendication 9, et/ou une cellule hôte selon la revendication 10, et,
b) ainsi, la transfection de manière stable d'une cellule hôte.

15. Utilisation d'un polynucléotide selon l'une quelconque des revendications 1 à 8 pour la modification génétique de manière stable d'une cellule hôte.
